# EUROPÄISCHE PATENTANMELDUNG

(11) **EP 2 026 148 A2**
(43) Veröffentlichungstag der Anmeldung: **18.02.2009**
(21) Anmeldenummer: 08075268.6
(22) Anmeldetag: 31.03.2008
(51) Int. Cl.: G05B 19/042

(54) **Verfahren zur sicheren Umprogrammierung klinisch relevanter Parameter im Rahmen der Fernprogrammierung eines elektronischen Implantates**

(30) Priorität: 11.08.2007 DE 102007037948
(71) Anmelder: BIOTRONIK CRM Patent AG, 6341 Baar (CH)
(72) Erfinder: Dörr, Thomas, 12437 Berlin (DE)
(74) Vertreter: Lindner-Vogt, Karin L.

(57) **Zusammenfassung**

Die Erfindung betrifft ein fernprogrammierbares persönliches Gerät, insbesondere ein programmierbares implantierbares medizinisches Gerät, beispielsweise einen Herzschrittmacher, einen Defibrillator, einen Kardioverter oder dergleichen. Außerdem betrifft die Erfindung eine Anordnung für die Fernprogrammierung eines solchen persönlichen medizinischen Gerätes und ein Verfahren zum Fernprogrammieren eines programmierbaren persönlichen Gerätes.

## Beschreibung

Die Erfindung betrifft ein fernprogrammierbares persönliches Gerät, insbesondere ein programmierbares implantierbares medizinisches Gerät, beispielsweise einen Herzschrittmacher, einen Defibrillator, einen Kardioverter oder dergleichen. Außerdem betrifft die Erfindung eine Anordnung für die Fernprogrammierung eines solchen persönlichen medizinischen Gerätes und ein Verfahren zum Fernprogrammieren eines programmierbaren persönlichen Gerätes.

In Zusammenhang mit Herzschrittmachern oder Defibrillatoren im Verbund mit einem Service Center ist es möglich jedoch hinlänglich bekannt, seitens eines Herzschrittmacher oder Defibrillators gewonnene, medizinische, physiologische oder Betriebsdaten zu dem zentralen Service Center zu übertragen, um diese Daten dort auszuwerten und über eine entsprechende Benutzerschnittstelle einem betreuenden Arzt zur Verfügung zu stellen.

Einige Funktionen solcher Implantate sind durch Software oder Firmware gesteuert und daher programmierbar. Hierfür besitzen die Implantate eine programmierbare Steuerung zum Steuern dieser Funktionen.

Es kommt immer wieder vor, dass nach anfänglicher Programmierung kurz vor, während oder nach der Implantation des Implantats weitere Programmierungen oder Umprogrammierungen wünschenswert sind, um das Implantat besser auf möglicherweise inzwischen veränderte Gesundheitszustände eines Patienten einstellen zu können oder um die Leistungsfähigkeit des Implantats anderweitig zu erhöhen. Häufig geschieht ein derartiges Programmieren oder Umprogrammieren dadurch, dass ein Arzt zu einem jeweiligen Implantat mit Hilfe eines Programmiergerätes eine kurzreichweitige, drahtlose Datenverbindung herstellt und das Implantat im Angesicht des Patienten programmiert.

Eine Programmierung oder Umprogrammierung des Implantats kann jedoch grundsätzlich auch aus der Ferne, beispielsweise über das zentrale Service Center, geschehen. Hierzu kann eine Datenverbindung zwischen dem Service Center und einem Patientengerät hergestellt werden. Das Patientengerät befindet sich üblicherweise in der Nähe des Patienten und dient quasi als Relaisstation zwischen dem Implantat und dem Service Center. Dazu besitzt das Patientengerät zwei unterschiedliche, bidirektionale Datenkommunikationsschnittstellen, auf der einen Seite eine bidirektionale Datenkommunikation mit dem Implantat und auf der anderen Seite eine bidirektionale Datenkommunikation mit dem Service Center zu ermöglichen. Die Datenkommunikationsschnittstelle für die Verbindung zwischen dem Patientengerät und dem Service Center kann dabei für eine drahtlose oder drahtgebundene Verbindung ausgebildet sein, beispielsweise über ein Mobiltelefonnetz oder über ein Festnetz.

Während die herkömmliche Programmierung eines Implantats mit Hilfe eines Programmiergerätes durch einen Arzt in Angesicht des Patienten erfolgt, sieht der Arzt den Patienten bei der Fernprogrammierung nicht. Der Arzt hat den Patienten bei der Fernprogrammierung nicht direkt vor Augen und kann daher nicht so leicht auf unmittelbare Äußerungen des Patienten reagieren.

Die Erfinder sind zu der Erkenntnis gelangt, dass dieser Umstand bei der Gestaltung des Implantats sowie der Anordnung zum Fernprogrammieren des Implantates berücksichtigt werden sollte.

Erfindungsgemäß wird hierzu ein programmierbares persönliches Gerät der eingangs genannten Art vorgeschlagen, das zwei Datenkommunikationsschnittstellen für eine drahtlose Datenübertragung von Daten zu und von dem programmierbaren persönlichen Gerät aufweist. Über beide Datenkommunikationsschnittstellen können insbesondere Programmieraufträge an das persönliche Gerät gesandt werden. Programmieraufträge sind Datenpakete, die Werte für Steuerparameter enthalten, die die Funktionsweise einer programmierbaren Steuerung des persönlichen Gerätes und damit das operative Verhalten des persönlichen Gerätes selbst bestimmen.

Die erste Datenkommunikationsschnittstelle dient der Nahprogrammierung des persönlichen Gerätes aus unmittelbarer Nähe beispielsweise mit einem herkömmlichen Programmiergerät. Wenn das persönliche Gerät ein Implantat ist, geschieht die Programmierung des persönlichen Gerätes über die erste Datenkommunikationsschnittstelle in Angesicht des Patienten.

Die zweite Datenkommunikationsschnittstelle dient der Fernprogrammierung des persönlichen Gerätes. Die erste und die zweite Datenkommunikationsschnittstelle unterscheiden sich beispielsweise hinsichtlich der für die Datenübertragung erforderlichen Übertragungstechnik, beispielsweise Modulation oder entsprechender elektromagnetischer Signale, oder durch das Datenformat.

Eine bereits erwähnte programmierbare Steuerung des programmierbaren Geräts ist ausgebildet, ein Steuern von Funktionen des persönlichen Gerätes anhand von Steuerparametern durchzuführen. Die Steuerparameter können verschiedene Parameterwerte annehmen, die zu jeweils unterschiedlichen Funktionen führen. Beispielsweise ist bei einem Herzschrittmacher die Stärke eines beispielsweise ventrikulären Stimulationsimpulses - angegeben in Volt oder Milliampere - ein Steuerparameter, der verschiedene Parameterwerte annehmen kann. Ein geeigneter Parameterwert für die Stärke eines ventrikulären Stimulationsimpulses wird üblicherweise mittels Reizschellentest durch den Arzt oder das Gerät selbst bestimmt. Andere Parameterwerte werden dem persönlichen Gerät durch Programmierung ohne vorherige Testung vorgegeben. Um eine derartige Programmierung zu ermöglichen, ist die programmierbare Steuerung sowohl mit der ersten als auch mit der zweiten Datenkommunikationsschnittstelle verbunden und kann über diese Programmieraufträge, die Parameterwerte für die Steuerparameter des persönlichen empfangen.

Um solche Parameterwerte speichern zu können, besitzt das persönliche Gerät einen Speicher, der mit der programmierbaren Steuerung verbunden ist.

Erfindungsgemäß sind die programmierbare Steuerung und der Speicher in Verbindung miteinander so ausgebildet, dass zu wenigstens einigen der von außen programmierbaren Steuerparameter des persönlichen Gerätes neben einem jeweils aktuellen Parameterwert wenigstens noch eine Kategoriekennzeichnung gespeichert werden kann. Die Kategoriekennzeichnung kennzeichnet die Zugehörigkeit des entsprechenden Steuerparameters zu wenigstens einer von wenigstens zwei Kategorien. Hierbei ist eine Kategorie von Steuerparametern vorgesehen, deren Parameterwerte nicht zunächst für eine kurze Zeit testweise eingestellt werden bevor sie dann dauerhaft bestätigt werden, sondern die von vornherein und ohne dass eine Reaktion des Patienten auf die Veränderung der Steuerparameter abgewartet werden muss, eingestellt werden. Die zweite Kategorie bezeichnet solche Steuerparameter, die nicht sofort dauerhaft programmiert werden sollen, sondern die zunächst für eine kurze Zeit getestet werden, um den Effekt der Umprogrammierung prüfen zu können, bevor diese Steuerparameter dann anschließend entweder wieder zurückgesetzt werden oder dauerhaft umprogrammiert werden.

Bezogen auf ein persönliches Gerät wie einen Herzschrittmacher oder Defibrillator zählen zu der ersten Kategorie von Steuerparametern diejenigen Steuerparameter, die auch bei einer Nachsorge mit direktem Patientenkontakt umprogrammiert werden können, ohne dass eine Kontrolle der neuen Parametereinstellungen während der Nachsorge möglich oder üblich ist. Die zweite Kategorie von Steuerparametern fasst alle Steuerparameter zusammen, die innerhalb einer Nachsorge hinsichtlich der Patientensicherheit vom Arzt nach Umprogrammierung geprüft werden müssen oder zunächst mittels eines Temporärprogramms getestet werden.

Die jeweiligen Steuerparametern zugeordnet gespeicherten Kategoriekennzeichnungen bestimmen das Verhalten der programmierbaren Steuerung nach Empfang eines Programmierauftrags über die zweite Datenkommunikationsschnittstelle, so dass
nach Ausführen eines Programmierauftrags, der ausschließlich zulässige Parameter Werte für Steuerparameter einer ersten Kategorie enthält, diese Parameter dauerhaft als aktuelle Parameterwerte in dem Speicher gespeichert werden, so dass fortan die Funktion der programmierbaren Steuerung bis zum Empfangen eines weiteren Programmierauftrages durch diese Parameterwerte bestimmt ist; und
nach Ausführen eines Programmierauftrags, der ausschließlich zulässige Parameterwerte für Steuerparameter der zweiten Kategorie enthält, diese Parameterwerte für eine vorgegebene, begrenzte Zeitdauer als aktuelle Parameterwerte in dem Speicher gespeichert werden, so dass die Funktion der programmierbaren Steuerung und damit des persönlichen Gerätes, bis zum Ende dieser vorgegebenen Zeitdauer durch diese Parameter bestimmt ist und anschließend wieder durch die ursprünglich eingestellten Parameterwerte bestimmt ist.

Vorzugsweise ist das persönliche Gerät durch entsprechendes Zusammenwirken von programmierbarer Steuerung und Speicher dazu ausgebildet, auch Programmieraufträge zu empfangen, die sowohl zulässige Parameter für Steuerparameter einer ersten als auch einer zweiten Kategorie enthalten.

Gemäß einer ersten Variante ist hierzu die programmierbare Steuerung derart ausgebildet, dass alle Parameter für eine vorgegebene, begrenzte Zeitdauer als aktuelle Parameterwerte in dem Speicher gespeichert werden, so dass die Funktion der programmierbaren Steuerung bis zum Ende der vorgegebenen Zeitdauer durch diese Parameterwerte bestimmt ist und anschließend wieder durch die zuvor eingestellten Parameterwerte. Das heißt, dass auch die grundsätzlich für eine dauerhafte Umprogrammierung von vorn herein vorgesehenen Steuerparameter zunächst nur temporär umprogrammiert werden, wenn sie zusammen mit Steuerparametern der zweiten Kategorie umprogrammiert werden. Hierdurch ist sichergestellt, dass alle gleichzeitig umprogrammierten Steuerparameter miteinander konsistent sind. Denn es ist möglich, dass die konkrete Wahl der ersten Steuerparameter auf die gleichzeitig umprogrammierten zweiten Steuerparameter abgestimmt ist.

Alternativ könnte die programmierbare Steuerung so ausgebildet sein, dass sie nach Empfang eines Programmierauftrags, der sowohl Parameterwerte für Steuerparameter der ersten Kategorie als auch der zweiten Kategorie enthält, die Parameterwerte der Steuerparameter der ersten Kategorie dauerhaft als aktuelle Parameterwerte in dem Speicher gespeichert werden, während die Parameterwerte der Steuerparameter der zweiten Kategorie zunächst nur für eine vorgegebene, begrenzte Zeitdauer als aktuelle Parameterwerte in dem Speicher gespeichert werden.

Darüber hinaus kann es für alle Fälle von Programmieraufträgen, die Steuerparameter der zweiten Kategorie enthalten, vorteilhaft sein, wenn das persönliche Gerät die Möglichkeit bietet, sich nach Empfang eines solchen Programmierauftrags in einem bestimmten Betriebsmodus zu versetzen. Dies kann bei einem persönlichen Gerät in Form eines Herzschrittmacher, Kardioverters oder Defibrillators beispielsweise ein an sich bekannter VVI-Modus sein, ein AAI-Modus oder ein DDD-Modus oder dergleichen.

Der Betriebszustand (Betriebsmodus) ist dabei jeweils so gewählt, dass er einen sicheren, insbesondere einen patientensicheren Betrieb des persönlichen Gerätes mit den entsprechenden Parameterwerten erlaubt.

Weiterhin ist es vorteilhaft, wenn die programmierbare Steuerung dazu ausgebildet ist, nach Ablauf der vorgegebenen Zeitdauer - also nach dem die Steuerparameter insbesondere der zweiten Kategorie getestet wurden - ein Datenpaket mit während der vorgegebenen Zeitdauer gewonnenen physiologischen oder Betriebsdaten über die erste oder die zweite Datenkommunikationsschnittstelle zu versenden. Ein derartiges Datenpaket kann dann beispielsweise von einem Service Center empfangen werden. Das Datenpaket kann dabei beispielsweise Daten enthalten, die ein intrakardiales Elektrokardiogramm repräsentieren, das während der vorgegebenen Zeitdauer - also während der temporären Umprogrammierung des persönlichen Gerätes - aufgenommen wurde und welches dann aus der Ferne von einem Arzt analysiert werden kann.

Insbesondere in Kombination mit der letztgenannten, bevorzugten Ausführungsvariante ist es vorteilhaft, wenn die programmierbare Steuerung weiterhin dazu ausgebildet ist, nach Ablauf der vorgegebenen Zeitdauer einen Programmierauftrag zu empfangen und auszuführen, der einen Steuerbefehl enthält, der eine dauerhafte Speicherung der Parameterwerte auch für die Steuerparameter der zweiten Kategorie in dem Speicher bewirkt. Damit kann ein Arzt, der beispielsweise ein seitens des persönlichen Gerätes empfangenes intrakardiales Elektrokardiogramm ausgewertet und festgestellt hat, dass die gewählten Parameterwerte der Steuerparameter insbesondere der zweiten Kategorie geeignet sind, diese Parameterwerte mit einem einfachen Programmierauftrag, der einen entsprechenden Steuerbefehl enthält, dauerhaft aktivieren.

Vorzugsweise ist das persönliche Gerät so ausgebildet, dass die das Verhalten des persönlichen Gerätes nach Empfangen eines Programmierauftrags entscheidenden Werte, insbesondere die Länge der vorgegebenen Zeitdauer für die temporäre Umprogrammierung des persönlichen Gerätes und die Kategoriekennzeichnungen, ebenfalls von außerhalb des persönlichen Gerätes programmierbar sind, und zwar konkret über die erste Datenkommunikationsschnittstelle für die Programmierung aus der Nähe.

Alternativ kann insbesondere die vorgegeben Zeitdauer auch Teil eines jeweiligen Programmierauftrags sein, der Parameterwerte für Steuerparameter der zweiten Kategorie enthält. Dann kann die Dauer der jeweiligen Aktivierung eines Parameterwertes der zweiten Kategorie mit demselben Programmierauftrag bestimmt werden, der auch den entsprechenden zu testenden Parameterwert enthält.

Wie bereits erwähnt, ist das persönliche Gerät vorzugsweise ein aktives medizinisches Implantat wie ein Herzschrittmacher oder Defibrillator/Kardioverter. Insbesondere in diesem Fall ist es vorteilhaft, wenn die zweite Datenkommunikationsschnittstelle für die Fernprogrammierung des persönlichen Gerätes eine drahtlose Kommunikation mit einer Reichweite im Bereich bis zu 5 m erlaubt und gemäß dem Medical Implant Communications Service Standard (MICS) ausgebildet ist.

Im Falle eines medizinischen Implantates ist ein Steuerparameter der ersten Kategorie vorzugsweise die Empfindlichkeit eines ventrikulären Eingangsverstärkers, während ein bevorzugter Steuerparameter der zweiten Kategorie eine VT Zonengrenze ist. Die VT bezeichnet eine Herzrate ab der das persönliche Gerät die Behandlung einer ventrikulären Tachykardie (VT) beginnt.

Erfindungsgemäß wird das eingangs genannte Ziel auch durch eine Anordnung zum Fernprogrammieren eines programmierbaren persönlichen Gerätes erreicht, dass neben einem persönlichen Gerät der zuvor beschriebenen Art auch noch wenigstens ein Servicecenter für die Programmierung des persönlichen Gerätes aus der Ferne aufweist. Das Servicecenter besitzt wenigstens eine Datenkommunikationsschnittstelle zum wenigstens mittelbaren Verbinden des Servicecenters mit dem persönlichen Gerät. Außerdem besitzt das Servicecenter einen Speicher und eine Programmiereinheit. Die Programmiereinheit ist so ausgebildet, dass sie in Kombination mit dem Speicher eine Benutzeroberfläche für das Fernprogrammieren des persönlichen Gerätes generieren kann, die ausgebildet ist, das Zusammenstellen eine Programmierauftrags für das persönliche Gerät zu erlauben. Hierzu werden vorzugsweise die fernprogrammierbaren Steuerparameter nebst ihrer Zugehörigkeit zu einer der beiden Kategorien auf der Benutzeroberfläche dargestellt. Zugänglich ist diese Benutzeroberfläche beispielsweise über einen an das Servicecenter angeschlossenen Computer oder auch über einen an das Servicecenter angeschlossenes Programmiergerät der herkömmlichen Art.

Vorzugsweise ist das Servicecenter so ausgebildet, dass es von dem persönlichen Gerät ausgesandte Datenpakete mit physiologischen Daten und/oder Betriebsdaten empfangen kann. Die Programmiereinheit ist in diesem Zusammenhang vorzugsweise so ausgebildet, dass sie diese Daten im Speicher des Servicecenter speichert.

Weiterhin ist die Programmiereinheit des Servicecenters gemäß einer bevorzugten Ausführungsvariante dazu ausgebildet, eine Benutzeroberfläche zu generieren, auf der die in dem Speicher des Servicecenters gespeicherten physiologischen Daten oder Betriebsdaten angezeigt werden.

Um schließlich einen zunächst nur temporär erteilten Programmierauftrag mit Parameterwerten für Steuerparameter der zweiten Kategorie derart zu bestätigen, dass das persönliche Gerät dauerhaft mit diesen Parameterwerten programmiert wird, ist die Programmiereinheit besonders bevorzugt dazu ausgebildet, eine Benutzeroberfläche zu generieren, dies erlaubt durch eine Benutzeraufgabe einen solchen Programmierauftrag zu bestätigen. Die Programmiereinheit ist dazu ausgebildet, daraufhin einen entsprechenden Programmierauftrag zu generieren und dann das persönliche Gerät abzusenden. Auf diese Weise erlaubt es die bevorzugte Variante des Servicecenters, Programmieraufträge leicht zusammen zu stellen, temporäre Programmieraufträge an Hand der vom persönlichen Gerät empfangenen Daten zu überprüfen und schließlich einen temporären Programmieraufträge so zu bestätigen (oder auch nicht), dass er dauerhaft wird.

Ein weiterer Beitrag zum Erreichen des eingangs genannten Ziels besteht in einem Verfahren zum Fernprogrammieren eines persönlichen Gerätes der zuvor beschriebenen Art mit einer zuvor beschriebenen Anordnung. Dieses Verfahren umfasst wenigstens die Schritte Senden eines Programmierauftrags von dem Servicecenter an das persönliche Gerät und anschließend als weiteren Verfahrensschritt entweder ein dauerhaftes Umprogrammieren des persönlichen Gerätes, wenn der Programmierauftrag nur Parameterwerte für Steuerparameter der ersten Kategorie enthält, oder temporäres Umprogrammieren des persönlichen Gerätes für eine vorgegebene Zeitdauer, wenn der Programmierauftrag Parameterwerte für Steuerparameter der zweiten Kategorie enthält, sowie anschließendes Zurückkehren zu den Ausgangswerten der zwischenzeitlich temporär umprogrammierten Parameterwerte.

Vorzugsweise umfasst das Verfahren die weiteren Verfahrensschritte.
- Erfassen von physiologischen Daten und/oder Betriebsdaten während der vorgegebenen Zeit und
- Senden eines physiologischen Daten und/oder Betriebsdaten enthaltenen Datenpaketes an das Servicecenter.

Darüber hinaus kann das Verfahren als weitere Verfahrensschritte enthalten:
- Bestätigen der Parameterwerte der Steuerparameter der zweiten Kategorie und dadurch dauerhaftes Umprogrammieren des persönlichen Gerätes mit den Parameterwerten der zweiten Kategorie.

Weitere vorteilhafte Ausgestaltungen der Erfindung ergeben sich des bisher nicht explizit genannten Kombinationen der bisher genannten Merkmale untereinander oder mit den weiteren Merkmalen, die sich aus den nachfolgenden Beschreibungen eines Ausführungsbeispiels ergeben.

Die Erfindung soll nun an Hand eines Ausführungsbeispiels mit Bezug auf die Figuren näher erläutert werden. Von den Figuren zeigt:
- Fig. 1:: eine Anordnung zum Fernprogrammieren eines persönlichen programmierbaren Gerätes;
- Fig. 2:: das persönliche programmierbare Gerät in detaillierterer Darstellung;
- Fig. 3:: den Ablauf einer Fernprogrammierung des persönlichen Gerätes mit einer Anordnung gemäß Figur 1 in einer ersten Variante;
- Fig. 4:: den Ablauf einer Fernprogrammierung des persönlichen Gerätes mit einer Anordnung gemäß Figur 1 in einer zweiten Variante; und
- Fig. 5:: den Ablauf einer Fernprogrammierung des persönlichen Gerätes mit einer Anordnung gemäß Figur 1 in einer dritten Variante.

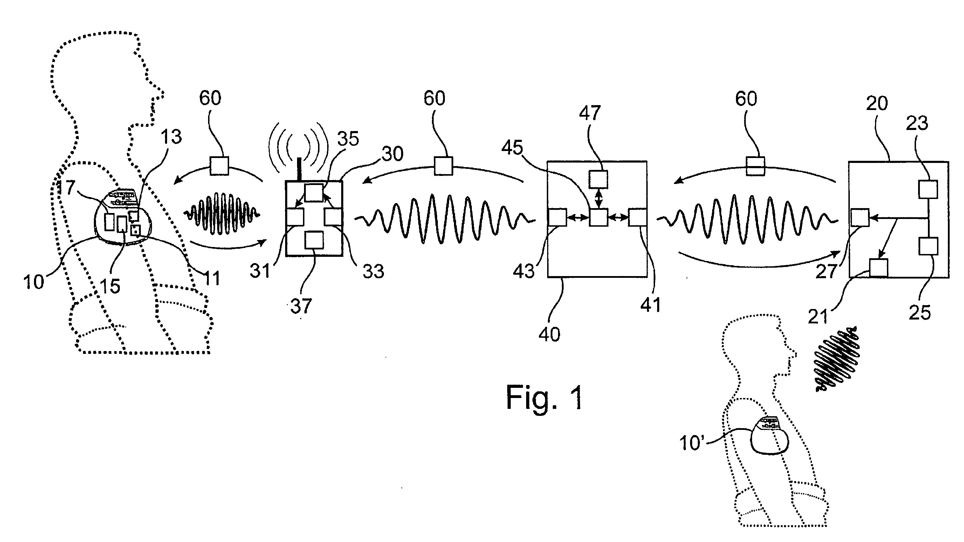

Die in Figur 1 dargestellte Anordnung zum Fernprogrammieren eines persönlichen Gerätes 10 umfasst neben dem persönlichen Gerät 10 in Form eines Herzschrittmachers ein Patientengerät 30, ein Servicecenter 40 sowie ein Programmiergerät 20.

Mit dem Programmiergerät 20 kann das Implantat 10- über eine sehr kurzreichweitige, drahtlose Datenverbindung direkt programmiert werden. Mit Hilfe des Patientengerätes 30 und des Servicecenters 40 kann das Implantat 10 auch aus der Ferne programmiert werden. Hierbei kann das Patientengerät 20 auch mit dem Servicecenter 40 verbunden sein, um beispielsweise einem mit Hilfe des Servicecenters 40 generierte Benutzeroberfläche für die Fernprogrammierung des Implantates 10 darzustellen.

Um sowohl direkt mit dem Programmiergerät 20 als auch aus der Ferne mit dem Service-center 40 bidirektional Daten austauschen zu können, sitzt das Patientengerät zwei Datenkommunikationsschnittstellen 11 und 13, von den eine erste Datenkommunikations-schnittstelle 11 eine kurzreichweitige Datenkommunikation direkt mit dem Programmiergerät 20 erlaubt.

Die zweite Datenkommunikationsschnittstelle 13 des Implantats 10 dient dazu, eine drahtlose Datenverbindung zwischen dem Implantat 10 und dem Patientengerät 30 herzustellen. Auch diese Datenverbindung ist nicht von besonders großer Reichweite. Üblicherweise wird sich das Patientengerät 30 immer in der Nähe des Implantats 10 befinden und dient dann als Station für eine weitere Datenübertragung von und zum Servicecenter 40.

Beide Datenkommunikationsschnittstelle 11 und 13 sind mit einer programmierbaren Steuerung 15 des Implantats verbunden, die wiederum mit einem Speicher 17 verbunden ist.

Das Patientengerät 30 besitzt ebenfalls eine erste Datenkommunikationsschnittstelle 31 für einen drahtlosen Datenaustausch mit dem Implantat 10 über dessen zweite Datenkommunikationsschnittstelle 13. Außerdem weist auch das Patientengerät 30 eine zweite Datenkommunikationsschnittstelle 33 auf, die dazu ausgebildet ist, eine Datenverbindung mit dem Servicecenter 40 herzustellen. Die erste und die zweite Datenkommunikations-schnittstelle 31 und 33 des Patientengerätes 30 sind wenigstens mittelbar über eine Steuereinheit 35 des Patientengerätes 30 miteinander verbunden. Außerdem weist das Patientengerät 30 eine eigenen Speicher 37 auf, in dem Daten - z.B. Programmieraufträge 60 - zwischengespeichert werden können falls diese beispielsweise seitens des Service Centers 40 empfangen wurden und noch nicht in das Implantat 10 weiter übertragen werden konnten.

Das Service Center 40 besitzt ebenfalls eine erste Datenkommunikationsschnittstelle 43, welche der Datenkommunikation mit dem Patienten 30 dient. Die Datenverbindung zwischen der zweiten Datenkommunikationsschnittstelle 33 des Patientengerätes 30 und der ersten Datenkommunikationsschnittstelle 43 des Service Centers 40 kann dabei drahtgebunden, beispielsweise über das Telefonfestnetz erfolgen.

Auch das Service Center 40 besitzt eine Steuereinheit 45 sowie einen mit diesem verbundenen Speicher 47. Die Steuereinheit 45 und die Speicher 47 stellen in Kombination miteinander unter anderem eine Programmiereinheit für das Fernprogrammieren des Implantats 10 dar. Insbesondere sind die Steuereinheit 45 und der Speicher 47 des Service Centers 40 so ausgebildet, dass sie eine Benutzeroberfläche für das Fernprogrammieren des Implantats 10 generieren können, die es einem Benutzer erlaubt, einen Programmierauftrag für das Implantat zusammenzustellen. Hierzu kann das Service Center 40 beispielsweise über das Internet mit einem Computer eines Arztes verbunden sein, auf dessen Bildschirmoberfläche dann die Benutzeroberfläche für das Fernprogrammieren des Implantats dargestellt wird.

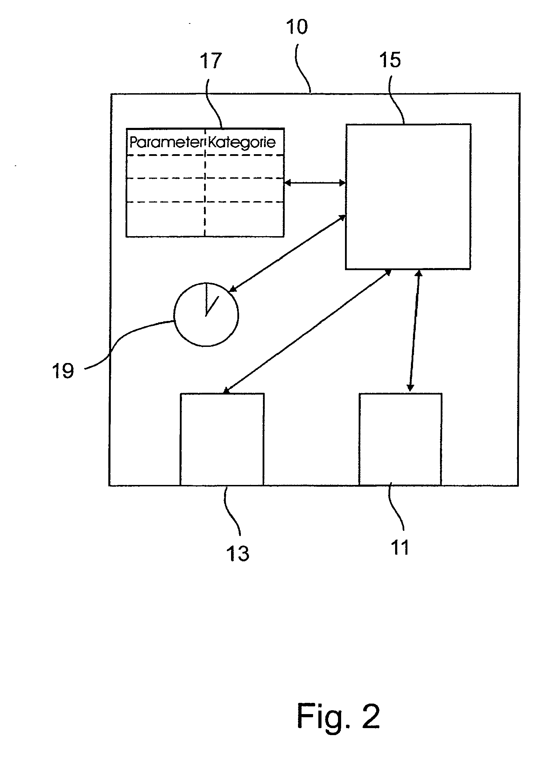

Wie insbesondere Fig. 2 zu entnehmen ist, ist der Speicher des Implantats 17 in Kombination mit der Steuerung 15 so organisiert, dass er neben aktuell für die Steuerung des Implantats benötigten Werten von Steuerparametern auch diesen Steuerparametern zugeordnete Kategoriekennzeichnungen speichert. Die in dem Speicher 17 gespeicherten Parameterwerte der Steuerparameter selbst bestimmen dabei die Funktionsweise der Steuerung 15 und damit des Implantats 10. Die Kategoriekennzeichnungen bestimmen das Verhalten des Implantats bei einer Fernprogrammierung, bei der das Implantat 10 einen Programmierauftrag 60 über die zweite Datenkommunikationsschnittstelle 13 empfängt.

Die in dem Speicher 17 gespeicherten Kategoriekennzeichnungen können nur bei einer direkten Datenverbindung zwischen dem Implantat 10' und dem Programmiergerät 20 verändert werden, sowie dies in Fig. 1 rechts unten dargestellt ist. Auf dem Wege der Fernprogrammierung können die Kategoriekennzeichnungen nicht verändert werden.

Für die Fernprogrammierung weist das Implantat 10 außerdem einen Zeitgeber 19 auf, der mit der programmierbaren Steuerung 15 verbunden ist und es dieser erlaubt, Steuerparameterwerte für eine durch den Zeitgeber vorgegebene Zeitdauer im Sinne eines empfangenen Programmierauftrags zu ändern und nach Ablauf der vorgegebenen Zeitdauer zu den ursprünglich herrschenden Parameterwerten zurückzukehren.

Das Verhalten des Implantats 10, welches durch die entsprechende Ausbildung der programmierbaren Steuerung 15 in Verbindung mit dem Speicher 17 bestimmt ist, soll nun näher erläutert werden.

Falls das Implantat 10 über die zweite Datenkommunikationsschnittstelle 13 einen Programmierauftrag 60 empfängt, der nur solche Parameterwerte enthält, die zu Steuerparametern einer ersten Kategorie, die im Folgenden mit Klasse 1 bezeichnet wird, gehören, werden diese Parameterwerte sofort an der entsprechenden Stelle in dem Speicher 17 dauerhaft gespeichert, sodass sie fortan die Funktionsweise des Implantats 10 bestimmen. Ob ein jeweiliger Parameterwert zu einem Steuerparameter der ersten oder der zweiten Kategorie zählt, ergibt sich für das Implantat 10 aus der entsprechenden Kategoriekennzeichnung, die zu einem jeweiligen Steuerparameter in dem Speicher 17 gespeichert ist.

Die Fernprogrammierung des Implantats 10 mit Parameterwerten zu Steuerparametern der Klasse 1 erfordert somit keine Rückmeldung an dem programmierenden Arzt.

Wenn ein Programmierauftrag Parameterwerte solcher Steuerparameter enthält, die zu einer mit Klasse 2 bezeichneten zweiten Kategorie zählen, werden zumindest diese Parameterwerte nur für eine begrenzte, vorgegebene Zeitdauer, die durch den Zeitgeber 19 bestimmt ist, in dem Sinne aktiviert, dass sie als diejenigen Parameterwerte gespeichert werden, die die Funktion des Implantats für die Dauer der vorgegebenen Zeitdauer bestimmen.

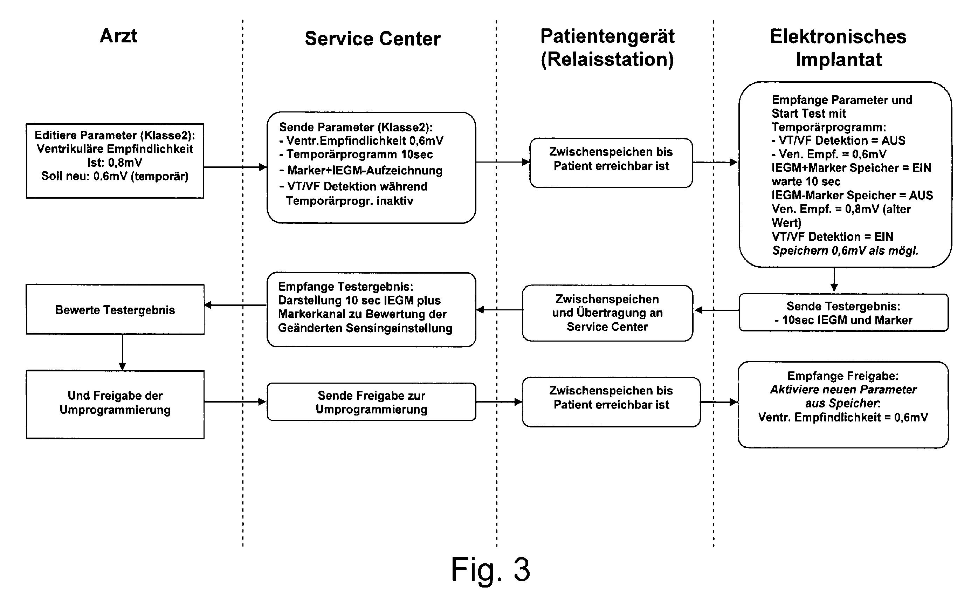

Konkrete Beispiele sind in Figuren 3 bis 5 dargestellt.

Figur 3 zeigt ein Beispiel, in dem ein Programmierauftrag ausschließlich Parameter für Steuerparameter der zweiten Kategorie (Klasse 2) enthält. Konkrete ist dies ein Parameterwert für den Steuerparameter "ventrikuläre Empfindlichkeit", der die Empfindlichkeit eines ventrikulären Eingangsverstärkers eines Herzschrittmachers definiert. Die Empfindlichkeit (Ansprechschwelle) vor der Programmierung ist 0,8 mV. Durch den Programmierauftrag soll die Empfindlichkeit erhöht werden, sodass die Ansprechschwelle bei 0,6 mV liegt.

Über eine vom Servicecenter generierte Benutzeroberfläche gibt der Arzt die gewünschte ventrikuläre Empfindlichkeit ein und das Servicecenter generiert daraus einen Programmierauftrag, der den neuen Wert für die ventrikuläre Empfindlichkeit und darüber hinaus die Dauer der vorgegebenen Zeitdauer enthält, nämlich 10 Sekunden. Darüber hinaus enthält der Programmierauftrag Steuerbefehle, die das Implantat veranlassen, während der 10 Sekunden ein intrakardiales Elektrokardiogramm (IEGM) aufzuzeichnen sowie Markensignale. Ein Markensignal gibt mit zeitlichem Bezug auf das intrakardiale Elektrokardiogramm an, wann beispielsweise eine ventrikuläre Kontraktion detektiert wurde, das heißt wann am Eingang des ventrikulären Eingangsverstärkers anliegendes Signal die Ansprechschwelle von 0,6 mV überschritten hat. Die erhöhte Empfindlichkeit des ventrikulären Eingangsverstärkers kann dazu führen, dass der ventrikuläre Erfassungskanal nicht nur auf originäre ventrikuläre Ereignisse, also ventrikuläre Kontraktionen, die durch eine R-Zacke im intrakardialen Elektrokardiogramm präsentiert sind, anspricht, sondern beispielsweise auch auf T-Wellen. Dann kann es passieren, dass die Erhöhung der ventrikulären Empfindlichkeit dazu führt, dass das Implantat eine vermeintliche Herzrate ermittelt, die als ventrikuläre Tachykardie (VT) oder als ventrikuläre Fibrillation (VF) detektiert wird. Um dieses zu vermeiden, wird die Detektion derartiger Tachykardien durch den Programmierauftrag ausgeschaltet.

Das Servicecenter sendet den entsprechenden Programmierauftrag über das Patientengerät an das Implantat.

Dort werden die mit dem Programmierauftrag empfangenen Parameterwerte und Steuerbefehle für die vorgegebene Dauer (10 Sekunden) aktiviert. Daraufhin erfasst das Implantat ventrikuläre Ereignisse mit einer Ansprechschwelle von 0,6 mV und generiert gleichzeitig ein intrakardiales Elektrokardiogramm (IEGM) mit Markensignalen, die anzeigen, wann die Ansprechschwelle von 0,6 mV überschritten wurde. Nach den 10 Sekunden versendet das Implantat das erfasste intrakardiale Elektrokardiogramm (IEGM) und die Markersignale in Form eines Datenpaketes über das Patientengerät an das Servicecenter. Gleichzeitig kehrt das Implantat zu den vor der temporären Umprogrammierung geltenden Parameterwerten und Steuerbefehlen zurück.

Das Servicecenter ist mit seiner Steuereinheit und seinem Speicher so ausgebildet, dass es aus dem von Seiten des Implantats empfangenen Datenpaket eine Benutzeroberfläche generiert, auf der das empfangene intrakardiale Elektrokadiengramm sowie die Markersignale für den Arzt dargestellt werden. Nach Betrachtung dieser Daten kann der Arzt entscheiden, ob er die Absenkung der Ansprechwelle für die ventrikuläre Empfindlichkeit dauerhaft machen will oder ob es dabei bleiben soll, dass das Implantat auf Basis der ursprünglich bestehenden Parameter weiter arbeitet.

Entscheidet sich der Arzt dazu, die Änderungen der Ansprechschwelle dauerhaft vorzunehmen, kann er dies dem Servicecenter durch ein sprechendes Bedienungselement auf der Benutzeroberfläche mitteilen. Das Servicecenter generiert daraufhin einen Programmierauftrag mit einem Steuerbefehl zur Freigabe der Umprogrammierung. Dieser Programmierauftrag wird über das Patientengerät dem Implantat übermittelt.

Das Implantat ist so ausgebildet, dass es auf einen derartigen Programmierbefehl hin die dauerhafte Umprogrammierung des Parameterwertes für die ventrikuläre Empfindlichkeit vornimmt. Dabei wird nur dieser Parameterwert geändert. Die Einstellung hinsichtlich der Detektion ventrikulärer Tachykardien oder ventrikulärer Fibrillationen so wie hinsichtlich des Aufzeichnens eines intrakardialen Elektrokadiengramms (IEGM) bleiben auf ihrem ursprünglichen Wert, das heißt die Detektion ventrikulärer Tachykardien oder Fibrillationen ist eingeschaltet und das Aufzeichnen intrakardialer Elektrokardiogramme ist nach Ablauf der 10 Sekunden ausgeschaltet. Damit ist die Umprogrammierung in dem Ausführungsbeispiel gemäß Figur 3 abgeschlossen.

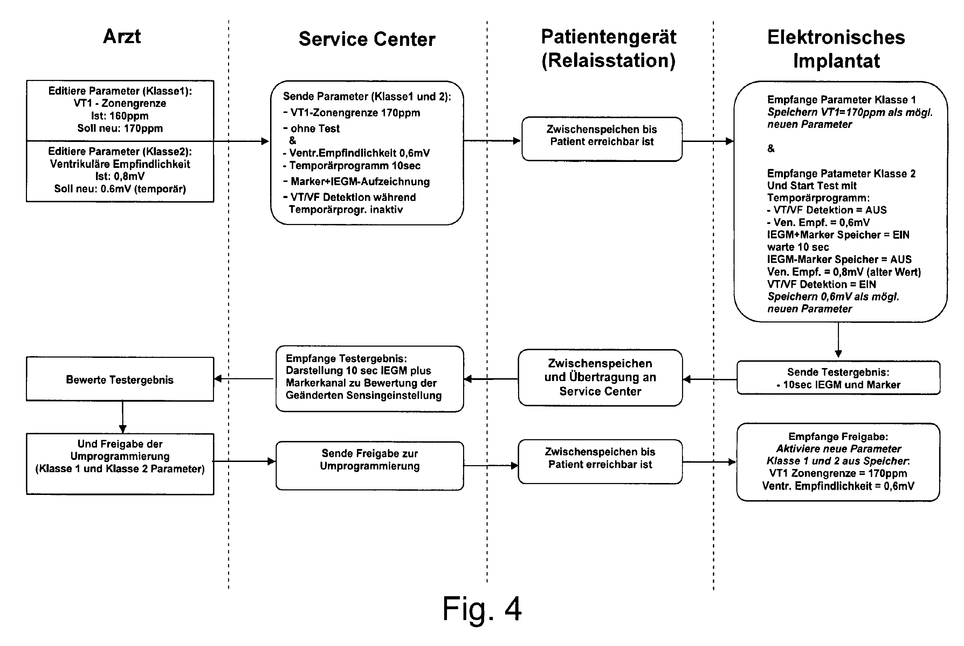

Figur 4 zeigt ein Ausführungsbeispiel, bei dem neben der zuvor bereits beschriebenen Umprogrammierung der ventrikulären Empfindlichkeit auch noch eine Zonengrenze für das Detektieren einer ventrikulären Tachykardie erster Ordnung geändert werden soll. Diese soll von 160 Schlägen pro Minute auf 170 Schläge pro Minute erhöht werden. Die VT1-Zonengrenze ist dabei ein Parameter der ersten Kategorie (Klasse 1) der grundsätzlich umprogrammiert werden kann, ohne dass es eine Rückmeldung an den Arzt bedarf, so das die Umprogrammierung sofort dauerhaft wirksam sein könnte.

Nach Eingabe der entsprechenden Werte über die Benutzeroberfläche generiert das Servicecenter einen entsprechenden Programmierauftrag und sendet diesen über das Patientengerät an das elektronische Implantat. In diesem wird temporär die Umprogrammierung der ventrikulären Empfindlichkeit vorgenommen. Dieser Parameterwert wird für 10 Sekunden aktiviert und anschließend wieder auf die ursprüngliche Empfindlichkeit zurückgestellt.
Wie im vorangegangenen Beispiel generiert das Implantat während der 10 Sekunden ein intrakardiales Elektrokardiogramm sowie Markersignale und sendet anschließend ein entsprechendes Datenpaket an das Servicecenter. Das Servicecenter veranlasst die automatische Darstellung dieser Daten auf einer Benutzeroberfläche, die es dem Arzt erlaubt, diese Daten zu begutachten. Daraufhin kann der Arzt wie zuvor beschrieben durch eine entsprechende Eingabe über die Benutzeroberfläche das Generieren eines Programmierauftrags veranlassen, der bewirkt, dass beide hinterlegten Änderungen der Parameterwerte, nämlich zum Einen für die ventrikuläre Empfindlichkeit und zum Anderen für die VT1-Zonengrenze, dauerhaft wirksam werden.

Nach Empfang dieses Programmierauftrags stellt das Implantat diese Parameterwerte als die fortan dauerhaft geltenden Parameterwerte ein.

Damit ist die Umprogrammierung gemäß dem Ausführungsbeispiel in Figur 4 abgeschlossen.

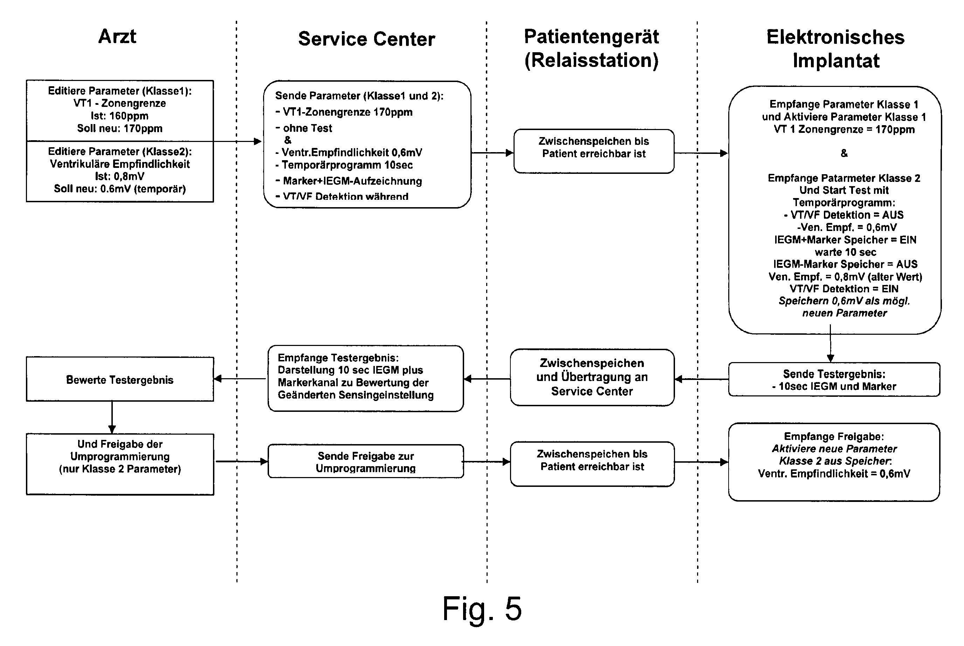

Figur 5 zeigt schließlich ein Ausführungsbeispiel welches dem aus Figur 4 sehr ähnlich ist.

Der Unterschied besteht darin, dass die Umprogrammierung des Steuerparameters der Kategorie 1 (in diesem Falle VT-1 Zonengrenze) sofort wirksam wird und nicht erst nach Bestätigung der geänderten Empfindlichkeit durch den Arzt.

Dementsprechend muss der Arzt auch nur noch die Umprogrammierung der ventrikulären Empfindlichkeit bestätigen. Ein entsprechender, vom Servicecenter zu generierender Programmierauftrag enthält daher einen Steuerbefehl, der die dauerhafte Umprogrammierung der ventrikulären Empfindlichkeit bewirkt. Die dauerhafte Umprogrammierung der VT-Zonengrenze ist bereits durch den ersten, zuvor ausgesandten Programmierauftrag bewirkt wurden.

## Patentansprüche

1. Programmierbares persönliches Gerät (10), insbesondere implantierbares medizinisches Geräte wie ein Herzschrittmacher, Kardioverter, Defibrillator oder dergleichen, mit den Komponenten:
erste Datenkommunikations-Schnittstelle (11) für eine drahtlose Datenübertragung, die so ausgebildet ist, dass das persönliche Gerät über die erste Datenkommunikations-Schnittstelle Programmieraufträge enthaltende Daten empfangen kann,
zweite Datenkommunikations-Schnittstelle (13) für eine drahtlose Datenübertragung, die so ausgebildet ist, dass das persönliche Gerät auch über die zweite Datenkommunikations-Schnittstelle (13) Programmieraufträge enthaltende Daten empfangen kann, wobei die erste und die zweite Datenkommunikations-Schnittstelle hinsichtlich des für die Datenübertragung erforderlichen Datenformats oder hinsichtlich der Datenübertragungstechnik unterschiedlich sind,
programmierbare Steuerung (15) für ein Steuern von Funktionen des persönlichen Gerätes anhand von Steuerparametern, wobei die programmierbare Steuerung (15) mit der ersten und der zweiten Datenkommunikations-Schnittstelle (11, 13) wenigstens mittelbar verbunden ist, und
Speicher (17), der der mit der programmierbaren Steuerung (15) verbunden ist,
wobei die programmierbare Steuerung (15) in Verbindung mit dem Speicher (17) ausgebildet ist, für jeden Steuerparameter jeweils wenigstens einen aktuellen Parameterwert und eine Kategoriekennzeichnung zu speichern, wobei die Kategoriekennzeichnung die Zugehörigkeit eines jeweiligen Steuerparameters zu wenigstens einer ersten oder einer zweiten Kategorie kennzeichnet und angibt, wie sich die programmierbare Steuerung (15) nach Empfang eines Programmierauftrags über die zweite Datenkommunikationsschnittstelle (13) verhält, und
wobei die programmierbare Steuerung (15) weiterhin ausgebildet ist,
nach Ausführen eines Programmierauftrags, der ausschließlich zulässige Parameterwerte für Steuerparameter einer ersten Kategorie enthält, diese Parameter dauerhaft als aktuelle Parameterwerte in dem Speicher (17) zu speichern, so dass die Funktion der programmierbaren Steuerung bis zum Empfang eines weiteren Programmierauftrags durch diese Parameterwerte bestimmt ist, oder
nach Ausführen eines Programmierauftrags, der ausschließlich zulässige Parameterwerte für Steuerparameter einer zweiten Kategorie enthält, diese Parameterwerte für eine vorgegebene, begrenzte Zeitdauer als aktuelle Parameterwerte in dem Speicher (17) zu speichern, so dass die Funktion der programmierbaren Steuerung (15) bis zum Ende der vorgegebenen Zeitdauer durch diese Parameterwerte bestimmt ist und anschließend wieder durch die zuvor eingestellten Parameterwerte.

2. Programmierbares persönliches Gerät gemäß Anspruch 1, **dadurch gekennzeichnet, dass** die programmierbare Steuerung dazu ausgebildet ist, nach Ausführen eines Programmierauftrags, der sowohl zulässige Parameterwerte für Steuerparameter einer ersten also auch einer zweiten Kategorie enthält, alle Parameterwerte für eine vorgegebene, begrenzte Zeitdauer als aktuelle Parameterwerte in dem Speicher zu speichern, so dass die Funktion der programmierbaren Steuerung bis zum Ende der vorgegebenen Zeitdauer durch diese Parameterwerte bestimmt ist und anschließend wieder durch die zuvor eingestellten Parameterwerte.

3. Programmierbares persönliches Gerät gemäß Anspruch 1, **dadurch gekennzeichnet, dass** die programmierbare Steuerung dazu ausgebildet ist, nach Ausführen eines Programmierauftrags, der sowohl zulässige Parameterwerte für Steuerparameter einer ersten also auch einer zweiten Kategorie enthält,
die Parameterwerte der Steuerparameter der ersten Kategorie dauerhaft als aktuelle Parameterwerte in dem Speicher zu speichern, so dass die Funktion der programmierbaren Steuerung bis zum Empfang eines weiteren Programmierauftrags durch diese Parameterwerte bestimmt ist und
die Parameterwerte der Steuerparameter der zweiten Kategorie für eine vorgegebene, begrenzte Zeitdauer als aktuelle Parameterwerte in dem Speicher zu speichern, so dass die Funktion der programmierbaren Steuerung bis zum Ende der vorgegebenen Zeitdauer durch diese Parameterwerte bestimmt ist und die Parameter der zweiten Kategorie anschließend wieder durch die zuvor eingestellten Parameterwerte.

4. Programmierbares persönliches Gerät gemäß wenigstens eines der Ansprüche 1 bis 3, **dadurch gekennzeichnet, dass** die programmierbare Steuerung dazu ausgebildet ist, nach Ausführen eines Programmierauftrags, der Parameterwerte für Steuerparameter der zweiten Kategorie enthält, das persönliche Gerät für die vorgegebene Zeitdauer in einen vorgegebenen Betriebszustand zu versetzen.

5. Programmierbares persönliches Gerät gemäß wenigstens eines der Ansprüche 1 bis 4, **dadurch gekennzeichnet, dass** die programmierbare Steuerung dazu ausgebildet ist, nach Ablauf der vorgegeben Zeitdauer ein Datenpaket mit während der vorgegebenen Zeitdauer gewonnenen physiologischen oder Betriebsdaten über die erste oder die zweite Datenkommunikations-Schnittstelle zu versenden.

6. Programmierbares persönliches Gerät gemäß wenigstens eines der Ansprüche 1 bis 5, **dadurch gekennzeichnet, dass** die programmierbare Steuerung dazu ausgebildet ist, nach Ablauf der vorgegebenen Zeitdauer einen Programmierauftrag zu empfangen und auszuführen, der einen Steuerbefehl enthält, der eine dauerhafte Speicherung der Parameterwerte auch für Steuerparameter der zweiten Kategorie in dem Speicher bewirkt.

7. Programmierbares persönliches Gerät gemäß eines der Ansprüche 1 bis 6, **dadurch gekennzeichnet, dass** die programmierbare Steuerung dazu ausgebildet ist, über die erste Datenkommunikations-Schnittstelle Programmieraufträge zu empfangen, die einen Wert für die vorgegebene Zeitdauer und/oder Kategoriekennzeichnungen für die Steuerparameter enthalten, und diesen Wert und/oder diese Kategoriekennzeichnungen in dem Speicher als von da an anzuwendenden Wert bzw. von da an anzuwendende Kategoriekennzeichnungen zu speichern.

8. Programmierbares persönliches Gerät gemäß wenigstens eines der Ansprüche 1 bis 7, **dadurch gekennzeichnet, dass** das persönliche Gerät ein aktives medizinisches Implantat ist.

9. Programmierbares persönliches Gerät gemäß wenigstens eines der Ansprüche 1 bis 8, **dadurch gekennzeichnet, dass** das persönliche Gerät ein implantierbarer Herzschrittmacher oder Defibrillator/Kardioverter ist.

10. Programmierbares persönliches Gerät gemäß einem der Ansprüche 1 bis 9, **dadurch gekennzeichnet, dass** die zweite Datenkommunikations-Schnittstelle des persönlichen Gerätes für eine drahtlose Kommunikation mit einer Reichweite im Bereich bis zu 5 m ausgebildet ist, insbesondere gemäß einer Medical Implant Communications Service-Spezifikation (MICS).

11. Programmierbares persönliches Gerät gemäß wenigstens eines der Ansprüche 8 bis 10, **dadurch gekennzeichnet, dass** Steuerparameter eine ventrikuläre Empfindlichkeit eines ventrikulären Eingangsverstärkers und eine VT-Zonengrenze, die einen Herzratenvergleichwert für eine Kardiovertersteuerung angibt, umfassen.

12. Programmierbares persönliches Gerät gemäß Anspruch 11, **dadurch gekennzeichnet, dass** die ventrikuläre Empfindlichkeit als Steuerparameter der ersten Kategorie **gekennzeichnet** ist und dass die VT-Zonengrenze als Steuerparameter der zweiten Kategorie **gekennzeichnet** ist..

13. Anordnung zum Fernprogrammieren eines programmierbaren persönlichen Gerätes, insbesondere eines implantierbaren medizinischen Gerätes wie einem Herzschrittmacher, Defibrillator oder dergleichen, mit den Komponenten:
- persönliches programmierbares Gerät (10) gemäß einem der Ansprüche 1 bis 10 und
- Service-Center (40) zur Programmierung des persönlichen Gerätes aus der Ferne,
wobei das Service-Center wenigstens
eine Datenkommunikations-Schnittstelle (43) zum wenigstens mittelbaren Verbinden des Servicecenters mit dem persönlichen Gerät,
einen Speicher (47), und
eine Programmiereinheit (45) für das persönliche Gerät aufweist, die ausgebildet ist, eine Benutzeroberfläche für das Fernprogrammieren des persönlichen Gerätes (10) so zu generieren, dass auf dieser für das Zusammenstellen eines Programmierauftrags für das persönliche programmierbare Gerät die für dieses persönliche Gerät programmierbaren Steuerparameter und mögliche Parameterwerte dargestellt werden.

14. Anordnung nach Anspruch 13, **dadurch gekennzeichnet, dass** das Servicecenter ausgebildet ist, vom persönlichen Gerät ausgesandte Datenpakte mit physiologischen und/oder Betriebsdaten zu empfangen und die Programmiereinheit ausgebildet ist, diese Daten in dem Speicher des Servicecenters zu speichern.

15. Anordnung nach Anspruch 14, **dadurch gekennzeichnet, dass** die Programmiereinheit ausgebildet ist, eine Benutzeroberfläche zu generieren, auf der die in dem Speicher des Servicecenters gespeicherten physiologischen Daten oder Betriebsdaten anzuzeigen.

16. Anordnung nach Anspruch 15, **dadurch gekennzeichnet, dass** Programmiereinheit ausgebildet ist, eine Benutzeroberfläche zu generieren, die es erlaubt durch eine Benutzereingabe einen Programmierauftrag, der Parameterwerte für Steuerparameter der zweiten Kategorie enthält, nach Ablauf der vorgegebenen Zeit zu bestätigen und daraufhin einen entsprechenden Programmierauftrag zu generieren und an das persönliche Gerät abzusenden.

17. Verfahren zum Fernprogrammieren eines programmierbaren persönlichen Gerätes gemäß einem der Ansprüche 1 bis 12, mittels einer Anordnung gemäß einem der Ansprüche 13 bis 16, mit den Verfahrensschritten:
- Senden eines Programmierauftrags von dem Servicecenter an das persönliche Gerät, anschließend
- entweder dauerhaftes Umprogrammieren des persönlichen Gerätes, wenn der Programmierauftrag nur Parameterwerte für Steuerparameter einer zweiten Kategorie enthält,
- oder temporäres Umprogrammieren des persönlichen Gerätes für eine vorgegebene Zeitdauer, wenn der Programmierauftrag Parameterwerte für Steuerparameter einer ersten Kategorie enthält und anschließendes Zurückkehren zu Ausgangswerten der temporär umprogrammierten Parameterwerte.

18. Verfahren nach Anspruch 17, **gekennzeichnet durch** die weiteren Verfahrensschritte:
- Erfassen von physiologischen Daten und/oder Betriebsdaten während der vorgegebenen Zeit und
- Senden eines die physiologischen Daten und/oder Betriebsdaten enthaltenden Datenpaketes an das Servicecenter.

19. Verfahren nach Anspruch 18, **gekennzeichnet durch** die zusätzlichen Verfahrensschritte:
- Bestätigen der Parameterwerte der Steuerparameter der zweiten Kategorie und **dadurch**
- dauerhaftes Umprogrammieren des persönlichen Gerätes mit den Parameterwerten der zweiten Kategorie.
